# EUROPÄISCHE PATENTSCHRIFT

(11) **EP 1 680 076 B1**
(45) Veröffentlichungstag und Bekanntmachung des Hinweises auf die Patenterteilung: **02.07.2008**
(21) Anmeldenummer: 04761875.6
(22) Anmeldetag: 23.08.2004
(51) Int. Cl.: A61K 8/34, A61K 8/37, A61Q 3/00, A61K 8/97, A61K 8/49, A61K 8/44, A61K 8/67

(54) **PHARMAZEUTISCHE UND KOSMETISCHE FORMULIERUNGEN ZUR BEHANDLUNG DER NÄGEL**
PHARMACEUTICAL AND COSMETIC FORMULATIONS FOR TREATING FINGERNAILS
FORMULATIONS PHARMACEUTIQUES ET COSMETIQUES POUR LE TRAITEMENT DES ONGLES

(30) Priorität: 25.08.2003 EP 03019157
(43) Veröffentlichungstag der Anmeldung: 19.07.2006
(73) Patentinhaber: BioEqual AG, 4132 Muttenz (CH)
(72) Erfinder: MEYER, Hans, CH-4125 Riehen (CH)
(74) Vertreter: Foraita, Hans-Günther
(86) Internationale Anmeldenummer: PCT/CH2004/000533
(87) Internationale Veröffentlichungsnummer: WO 2005/018585

(56) Entgegenhaltungen:
- EP-A- 0 179 675
- EP-A- 0 503 988
- EP-A- 1 475 072
- WO-A-02/00176
- WO-A-02/083084
- WO-A-03/045339
- WO-A1-97/34644
- CA-A- 1 072 009
- CH-A- 299 534
- DE-A- 10 014 673
- DE-A- 10 126 501
- US-B1- 6 361 785
- MERTIN, DIRK ET AL: "In-vitro permeability of the human nail and of a keratin membrane from bovine hooves: penetration of chloramphenicol from lipophilic vehicles and a nail lacquer" JOURNAL OF PHARMACY AND PHARMACOLOGY , 49(3), 241-245 CODEN: JPPMAB; ISSN: 0022-3573, 1997, XP009041391
- DATABASE WPI Section Ch, Week 198816 Derwent Publications Ltd., London, GB; Class D21, AN 1988-110264 XP002310735 & SU 1 337 095 A (BIOLAR IND COMPLEX) 15. September 1987 (1987-09-15)
- CHANTASART, DOUNGDAW ET AL: "Mechanistic studies of branched -chain alkanols as skin permeation enhancers" JOURNAL OF PHARMACEUTICAL SCIENCES , 93(3), 762-779 CODEN: JPMSAE; ISSN: 0022-3549, 2004, XP002310734

## Beschreibung

Die vorliegende Erfindung betrifft topisch anwendbare Mittel zur Behandlung von Nagelerkrankungen und zur Nagelpflege mit verbessertem Penetrationsvermögen durch die Nagelsubstanz und durch die Haut und beansprucht die Verwendung von Acetaten und Formiaten der Formel (I) gemäß den Ansprüchen 1 und 2.

Die direkte topische Behandlung von Nagelerkrankungen und die Nagelpflege verlaufen praktisch nebenwirkungsfrei, sind sehr einfach durchzuführen und verursachen nur geringe Kosten. Das wesentliche Problem der direkten topischen Anwendung von Nagelzubereitungen besteht jedoch darin, die Wirkstoffe, einschliesslich Nähr- und Aufbaustoffe, in ausreichender Menge durch den Nagel hindurch in die tiefer gelegenen Gewebeschichten und in die Nagelwurzel zu bringen und die dort vorhandenen Erreger vollständig abzutöten respektive den Nagel mit Nähr- und Aufbaustoffen zu versorgen. Mit herkömmlichen Mitteln gelingt es zwar, die Symptome durch direkte topische Behandlung zum Abklingen zu bringen, sie treten jedoch nach Abbruch der Behandlung in den meisten Fällen wieder auf.

Es wurde bereits vorgeschlagen, den Behandlungserfolg bei der direkten topischen Anwendung von Wirkstoffen dadurch zu verbessern, dass man die Wirkstoffe zusammen mit einem so genannten Carrier, d. h. einer Substanz, die neben einem guten Lösungsvermögen für den Wirkstoff auch ein gutes Penetrationsvermögen durch die Nagelsubstanz und die Fähigkeit besitzt, die Wirksubstanz durch das Nagelgewebe zu transportieren, zur Anwendung brachte. So werden beispielsweise in der EP-A-0 503 988 Arzneimittel zur Behandlung von Onychomykosen beschrieben, die neben einer mindestens teilweise in Wasser löslichen antimykotischen Wirksubstanz und einem aus einem geradkettigen oder verzweigten C₂-C₈Alkanol und zu mindestens einem Drittel aus Wasser bestehenden lösenden Medium mindestens eine hydrophile, die Penetration des Antimykotikums durch den Nagel fördernde Substanz enthalten. Als penetrationsfördernde Substanzen werden speziell Glykole, Monoäther von Glykolen, Diether von Glykolen, Dimethylsulfoxid, Caprolactam, Dimethylisosorbid, Isopropylidenglycerin, Dimethylimidazolidinon, N-Methylpyrrolidon-2, Pyrrolidon-2, Ethyllactat, Glyceride von C₈-C₁₀ Polyoxyethylenen, Polyethylenglykolglyceryllaurat und Dimethylacetamid genannt. Dieses in EP-A-0 503 988 beschriebene Formulierungsprinzip ist wegen der für die Wirksubstanz postulierten partiellen Wasserlöslichkeit nur für eine beschränkte Zahl von Wirksubstanzen, d.h. für viele Wirksubstanzen nicht anwendbar.

In der WO-A-97 34644 werden topische Formulierungen zur Behandlung von Nagel-Psoriasis, enthaltend n-Octanol als penetrationsförderndes Mittel offenbart.
In der publizierten WO-A-02 083084 werden Nagelbehandlungsmittel enthaltend Fluconazol, einen Carrier und Penetrationsverbesserer, insbesondere Caprylalkohol, t-Amylalkohol bzw. 3-Pentanol beschrieben..
In der Publikation von Mertin, Dirk et al. in Journal of Pharmacy and Pharmacology 49(3), 241-245 (1997) wird eine Zusammensetzung enthaltend Chloramphenicol und n-Octanol und erforderlichen Zusätzen auschliesslich zur Lackherstellung offenbart..
Die Anmeldung WO-A-03 045339 offenbart einen Lack als topisches Mittel zur Behandlung von Mykosen und Bakterien, wobei der Lack, enthaltend den Wirkstoff nicht den Nagel bzw. alle keratinhaltige Schichten durchdringt. Die DOS 10014673 beschreibt ein antimykotisches Nagelbehandlungsmittel in Form eines Lackes mit pflanzlichen Wirkstoffen und Amylacetat. Die DOS 101 26501 offenbart keratinablösende Zubereitungen enthaltend Harnstoff und niedere Alkanole.. Ferner ist die Anwesenheit von Wasser erforderlich.
Weitere Publikationen wie zum Beispiel das Canadische Patent 1072009 unterscheiden sich vorwiegend durch verschiedene Lösungs- bzw. Penetrationsverbesserer.
In der EP-A-0179 675 bzw. WO-A-0200176 wird die Herstellung von Lacken, Fettlacken und Salben beschrieben.

Die Anmeldung SU-A-1 337 095 (vgl. AN 1988-110264, Derwent Publications Ltd., London, GB; Section Ch, Week 198816, Class D21) offenbart einen Nagellackentferner mit Isoamylacetat und einer pflegend wirkenden Lipid-Kombination.
Die am 10.11.2004 veröffentlichte europäische Anmeldung EP-A-1 475 072 beansprucht die Priorität vom 24.12.2002 und beschreibt Mittel zur Nagelpflege, die als Wirkstoff zur Förderung des Wachstums der Nägel ein Dialkylsulfon enthalten.
Außerdem können die Mittel Lösungsmittel wie Isoamylacetat enthalten. Die Patentschrift CH-A-299 534 offenbart Nagellackzubereitungen enthaltend Amylformiat oder Amylacetat zusammen mit Pigmenten und Parfümkomponenten.

Keine der genannten Entgegenhaltungen offenbart die Verwendug von Formiaten oder Acetaten der Formel I als Penetrationsbeschleuniger für Wirkstoffe, die auf Nägeln angewendet werden.

Es gibt bisher kein zufrieden stellendes Mittel für die Verwendung zur topischen Behandlung von Nagelerkrankungen oder zur topischen Nagelpflege, das einen Carrier enthält, der den Transport einer für einen dauerhaften Behandlungserfolg erforderlichen Menge an Wirksubstanz durch den Nagel in das darunter liegende Nagelbett und zur Nagelwurzel (Matrix) gewährleistet.

Es ist daher die Aufgabe der vorliegenden Erfindung, die im Zusammenhang mit der Verwendung zur topischen Behandlung von Nagelerkrankungen und der topischen Nagelpflege bestehenden Probleme zu lösen und Mittel bereitzustellen, welche einen raschen und dauerhaften Behandlungserfolg ermöglichen.

Es wurde gefunden, dass Verbindungen der Formel I

R-O-R₁ (I)

in welcher
- R: einen geradkettigen oder verzweigten Alkylrest mit 5 - 8 Kohlenstoffatomen, und
- R₁: eine Formylgruppe oder eine Acetylgruppe bedeutet,
nicht nur ein ausgezeichnetes Penetrationsvermögen durch die keratinisierte Nagelsubstanz und durch die angrenzende Haut besitzen, sondern dass sie darüber hinaus die Fähigkeit haben, sowohl therapeutische Wirksubstanzen, wie Antimykotika, Antibiotika, Antiseptika und Kortikosteroide, als auch zur Nagelpflege geeignete Substanzen, wie Nähr- und Aufbaustoffe, durch die keratinisierte Nagelsubstanz und durch die Haut zu transportieren.

Gegenstand der vorliegenden Erfindung ist daher
- die Verwendung von Verbindungen der Formel I zur Verbesserung des Penetrationsvermögens von pflegenden Wirksubstanzen bei Anwendung in Form eines topischen Mittels zur Nagelpflege,
- sowie die Verwendung von Verbindungen der Formel I zur Herstellung eines topisch anwendbaren Mittels zur Behandlung von Nagelerkrankungen, wie in den vorliegenden Ansprüchen definiert.

Die obige Formel I umfasst die Formiate und Acetate der unverzweigten primären und sekundären C₅-C₈Alkanole sowie die Formiate und Acetate der verzweigten Isomeren dieser Alkanole. Einzelne Vertreter von Formiaten und Acetaten von C₅-C₈Alkanolen der Formel I sind Amylformiat, Amylacetat, Isoamylformiat, Isoamylacetat, 1-Hexylformiat, 1-Hexylacetat, 2-Hexylformiat, 2-Hexylacetat, 1-Heptylformiat, 1-Heptylacetat, 2-Heptylformiat, 2-Heptylacetat, 1-Oktylformiat, 1-Oktylacetat, 2-Oktylformiat und 2-Oktylacetat. Bevorzugte C₅-C₈Alkylester sind C₅-C₈Alkylacetate. Besonders bevorzugt sind C₅-C₆Alkylacetate. Weiterhin bevorzugt sind Mischungen von mehrreren C₅-C₆Alkylacetaten.

Für die erfindungsgemässen topisch anwendbaren Mittel kommen grundsätzlich alle therapeutischen Wirksubstanzen synthetischer und natürlicher Herkunft in Betracht, die bei Nagel- und Periungual-Erkrankungen wirksam sind. Ferner kommen als Wirksubstanzen Nähr- und Aufbaustoffe in Betracht, die bei der Nagelpflege wirksam sind.

Geeignete therapeutische Wirksubstanzen, die in den erfindungsgemässen Mitteln zur topischen Behandlung von Nagelerkrankungen enthalten sein können, sind Antimykotika synthetischer und natürlicher Herkunft, Antibiotika, Antiseptika und Kortikosteroide, sowie Kombinationen der genannten Wirksubstanzen.

Besonders geeignete Wirkstoffe sind Antimykotika synthetischer und natürlicher Herkunft und Nähr- und Aufbaustoffe, die bei der Nagelpflege wirksam sind.

Spezielle Beispiele für therapeutische Wirksubstanzen sind:
- Antimykotika und ihre physiologisch verträglichen Salze, wie z.B. (±)-cis-2,6-Dimethyl-4-[2-methyl-3-(*p-tert*-pentylphenyl)propyl]morpholin (Amorolfin), Amphotericin, 6-Cyclohexyl-1-hydroxy-4-methyl-2(1H)-pyridinon (Ciclopirox), Bis-phenyl-(2-chlorphenyl)-1-imidazolylmethan (Clotrimazol), 1-[2-(2,4-Dichlorphenyl)-2-(4-chlorbenzyloxy)-ethyl]-imidazol (Econazol), 2,4-Difluor-α,α-bis(1*H*-1,2,4-triazol-1-ylmethyl)benzylalkohol (Fluconazol), 5-Fluorcytosin (Flucytosin), 7-Chlor-trimethoxy-methylspiro-[benzofuran-cyclohexen]-dion (Griseofulvin), 1-[2,4-Dichlor-β-(2,6-dichlorbenzyloxy)-phenethyl]-imidazol (Isoconazol), (±)-1-*sec*-Butyl-4-{4-[4-(4-{[(2R*,4S*)-2-(2,4-dichlorphenyl)-2-(1,2,4-triazol-1-ylmethyl)-1,3-dioxolan-4-yl]methoxy}phenyl)-1-piperazinyl]phenyl}-4,5-dihydro-1,2,4-triazol-5-on (Itraconazol), (±)-cis-1-Acetyl-4-{4-([2-(2,4-dichlorphenyl)-2-(1H-imidazol-1-ylmethyl)-1,3-dioxolan-4-yl]methoxy)phenyl}piperazin (Ketoconazol), 1-[2,4-Dichlor-β-(2,4-dichlorbenzyloxyl)-phenethyl]-imidazol (Miconazol), (*E*)-N-Cinnamyl-*N*-methyl-1-naphthylmethylamin (Naftifin), Nystatin, (*E*)-*N-*(6,6-Dimethyl-2-hepten-4-inyl)-*N*-methyl-1-naphthylmethylamin (Terbinafin), 1[2-{(2-Chlor-3-thienyl)methoxy}-2-(2,4-dichlorphenyl)ethyl]-1H-imidazol (Tioconazol) und O-2-Naphthyl-N-methyl-N-(3-tolyl)-thiocarbamat (Tolnaftat).
   Gemäss vorliegender Erfindung bevorzugte Antimykotika sind (±)-cis-2,6-Dimethyl-4-[2-methyl-3-(*p-tert*-pentyl-phenyl)propyl]morpholin (Amorolfin), Bis-phenyl-(2-chlorphenyl)-1-imidazolylmethan (Clotrimazol), 1-[2,4-Dichlor-β-(2,6-dichlorbenzyloxy)-phenethyl]-imidazol (Isoconazol), 2,4-Difluor-α,α-bis(1*H*-1,2,4-triazol-1-ylmethyl)benzylalkohol (Fluconazol), (±)-1-*sec*-Butyl-4-{4-[4-(4-{[(2R*,4S*)-2-(2,4-dichlorphenyl)-2-(1,2,4-triazol-1-ylmethyl)-1,3-dioxolan-4-yl]methoxy}phenyl)-1-piperazinyl]phenyl}-4,5-dihydro-1,2,4-triazol-5-on (Itraconazol), (±)-cis-1-Acetyl-4-{4-([2-(2,4-dichlorphenyl)-2-(1H-imidazol-1-ylmethyl)-1,3-dioxolan-4-yl]methoxy)phenyl}piperazin (Ketoconazol), 1-[2,4-Dichlor-β-(2,4-dichlorbenzyloxyl)-phenethyl]-imidazol (Miconazol), (*E*)*-N-*(6,6-Dimethyl-2-hepten-4-inyl)-*N*-methyl-1-naphthylmethylamin (Terbinafin), α-(2,4-Difluorophenyl)-5-fluoro-β-methyl-α-(1H-1,2,4-triazol-1-ylmethyl)-4-pyrimidinethanol (Voriconazol).
   Gemäss vorliegender Erfindung besonders bevorzugte Antimykotika sind (±)-cis-2,6-Dimethyl-4-[2-methyl-3-(*p-tert*-pentylphenyl)propyl]morpholin (Amorolfin), Bis-phenyl-(2-chlorphenyl)-1-imidazolylmethan (Clotrimazol), 1-[2,4-Dichlor-β-(2,6-dichlorbenzyloxy)-phenethyl]-imidazol (Isoconazol), (±)-1-*sec*-Butyl-4-{4-[4-(4-{[(2R*,4S*)-2-(2,4-dichlorphenyl)-2-(1,2,4-triazol-1-ylmethyl)-1,3-dioxolan-4-yl]methoxy}phenyl)-1-piperazinyl]phenyl}-4,5-dihydro-1,2,4-triazol-5-on (Itraconazol), (±)-cis-1-Acetyl-4-{4-([2-(2,4-dichlorphenyl)-2-(1H-imidazol-1-ylmethyl)-1,3-dioxolan-4-yl]methoxy)phenyl}piperazin (Ketoconazol).
- Antimykotika natürlicher Herkunft, wie beispielsweise ätherische Öle und Pflanzenextrakte.
   Bevorzugte Antimykotika natürlicher Herkunft sind Teebaumöl (Melaleuca alternifolia), Lavendelöl (Lavandula officinalis chaix), Australian Blue Cypress Oil (callitis intratropica) und Blattextrakt des Niembaumes (Azadirachta indica). Diese natürlichen Antimykotika können als einzelne Wirkstoffe oder als Kombination mehrerer solcher Wirkstoffe verwendet werden. Eine bevorzugte Wirkstoffkombination ist eine Mischung von Lavendelöl, Teebaumöl und Australian Blue Cypress Oil.
- Antibiotika und ihre physiologisch verträglichen Salze, wie z.B. α-Amino-4-hydroxybenzylpenicillin (Amoxicillin), D-(-)-α-Aminobenzylpenicillin (Ampicillin), 3,3-Dimethyl-7-oxo-6-phenylacetamido-4-thia-1-azabicyclo-[3.2.0]-heptan-2-carbonsäure (Benzylpenicillin), Benzylpenicillin-Benzathin, 3-Chloro-7-D(2-phenylglycinamido)-cephalosporansäure (Cefaclor), 7β-[D-2-Amino-(4-hydroxy-phenyl)-acetylamino]-3-methyl-cephalosporansäure (Cefadroxil), Aminophenyl-acetamido-methyl-cephalosporansäure (Cefalexin), D(-)-threo-2-dichloracet-amido-1-(4-nitrophenyl)-1,3-propandiol (Chloramphenicol), 1-Cyclopropyl-6-fluor-1,4-dihydro-4-oxo-7-(1-piperazinyl)-3-chinolincarbonsäure (Ciprofloxacin), (Z)-(2R,5R)-3-(2-Hydroxyethyliden)-7-oxo-4-oxa-1-azabicyclo[3.2.0]heptan-2-carbonsäure (Clavulansäure), 7-Chlor-7-desoxy-lincomycin (Clindamycin), 6-Desoxy-5-hydroxytetracyclin (Doxycyclin), 1-Ethyl-6-fluor-1,4-dihydro-4-oxo-7-(1-piperazinyl)-1,8-naphthyridin-3-carbonsäure (Enoxacin), Erythromycin, 3-(2-Chlor-6-fluorphenyl)-5-methyl-4-isoxazolyl-penicillin (Flucloxacillin), Kanamycin, Lincomycin, 7-Dimethylamino-6-desoxy-6-desmethyltetracyclin (Minocyclin), 6-(2-ethoxy-1-naphthamido)-penicillin (Nafcillin), 1-Ethyl-1,4-dihydro-7-methyl-4-oxo-1,8-naphthyridin-3-carbonsäure (Nalidixinsäure), Neomycin, 1-Ethyl-6-fluoro-1,4-dihydro-4-oxo-7-(1-piperazinyl)-3-chinolincarboxylsäure (Norfloxacin), (±)-9-Fluor-2,3-dihydro-3-methyl-10-(4-methyl-1-piperazinyl)-7-oxo-7*H*-pyrido[1,2,3-*de*][1,4]benzoxazin-6-carbonsäure (Ofloxacin), 6-(5-Methyl-3-phenyl-4-isoxazolcarboxamido)penicillansäure (Oxacillin), 6-Phenoxyacetylamino-penicillansäure (Phenoxymethylpenicillin), 4-Dimethylamino-octahydro-pentahydroxy-1,11-dioxo-6-methylnaphtacen-2-carbamid (Tetracyclin).
   Bevorzugte Antibiotika sind Doxycyclin, Minocyclin, Neomycin. Antiseptika, wie z.B. Alkylbenzyldimethylammoniumchlorid (Benzalkonium-chlorid), N-Benzyl-N,N-dimethyl-2-{2-[p-(1,1,3,3,-tetramethylbutyl)-phenoxy]-ethoxy}-ethylammoniumchlorid (Benzethoniumchlorid), Cetyltrimethylammoniumbromid (Cetrimoniumbromid), 1,1'-Hexamethylen-bis-[5-(p-chlorphenyl)-biguanid] (Chlorhexidin), 1,10 -Dekamethylen-bis-(4-amino-2-methylclionolinium)-dichlorid (Dequaliniumchlorid), N-(4-Chlorphenyl)-N'-(3,4-dichlorphenyl)harnstoff (Triclocarban), 5-Chlor-2-(2,4-dichlorphenoxy)-phenol (Triclosan).
   Bevorzugte Antiseptika sind. 1,1'-Hexamethylen-bis-[5-(p-chlorphenyl)-biguanid] (Chlorhexidin).
- Kortikosteroide und ihre physiologisch verträglichen Salze, wie z.B. 9α-Chlor-16β-methylprednisolon (Beclomethason), 9-Fluor-11 β,17,21-trihydroxy-16β-methyl-1,4-pregnadien-3,20-dion (Betamethason), 21-Chlor-9-fluor-11β,17-dihydroxy-16β-methyl-1,4-pregnadien-3,20-dion (Clobetasol), 17,21-Dihydroxy-pregn-4-en-3,11,20-trion (Cortison), 11β,16α,17α,21-Tetrahydroxy-1,4-pregnadien-3,20-dion-16,17-acetonacetal (Desonid), 9-Fluor-11β-17,21-trihydroxy-16α-methylpregna-1,4-dien-3,20-dion (Dexamethason), 9α,11β-Dichloro-6α-fluoro-21-hydroxy-16α,17α-(isopropylidendioxy)-pregna-1,4-dien-3,20-dion (Flucloronid), 6α,9α-Difluor-16α,17α-isopropylidendioxy-corticosteron (Fluocinolonacetonid), 6α,9α-Difluor-16α,17α-isopropylidendioxy-corticosteron-acetat (Fluocinonid), 6α-Fluor-11β,21-dihydroxy-16α,17-isopropylidendioxy-4-pregnen-3,20-dion (Fludroxycortid), 3-(2-chloroethoxy)-9α-fluoro-6-formyl-11β,21-dihydroxy-16α,17α-isopropylidendioxypregna-3,5-dien-20-on (Formocortal), 21-Chlor-9α-fluor-11β-hydroxy-16α,17α-isopropylidendioxy-4-pregnen-3,20-dion (Halcinonid), 17α-Hydroxycorticosteron (Hydrocortison), 11β,17,21-Trihydroxy-6α-methyl-1,4-pregnadien-3,20-dion (Methylprednisolon), 11β,17,21-Trihydroxy-pregna-1,4-dien-3,20-dion (Prednisolon), 17α,21-Dihydroxy-pregna-1,4-dien-3,11,20-trion (Prednison), 9-Fluor-16a-hydroxyprednisolon (Triamcinolon), Triamcinolon-16α, 17α-acetonid (Triamcinolonacetonid).
   Bevorzugte Kortikosteroide sind 11β,16α,17α,21-Tetrahydroxy-1,4-pregnadien-3,20-dion-16,17-acetonacetal (Desonid), 9α,11β-Dichloro-6α-fluoro-21-hydroxy-16α,17α-(isopropylidendioxy)-pregna-1,4-dien-3,20-dion (Flucloronid), 6α,9α-Difluor-16α,17α-isopropylidendioxy-corticosteron (Fluocinolonacetonid), 6α,9α-Difluor-16a, 17α-isopropylidendioxy-corticosteron-acetat (Fluocinonid), 6α-Fluor-11β,21 -dihydroxy-16α, 17-isopropylidendioxy-4-pregnen-3,20-dion (Fludroxycortid), 3-(2-chloroethoxy)-9α-fluoro-6-formyl-11β,21-dihydroxy-16α,17α-isopropylidendioxypregna-3,5-dien-20-on (Formocortal), 21-Chlor-9α-fluor-11β-hydroxy-16α, 17α-isopropylidendioxy-4-pregnen-3,20-dion (Halcinonid), Triamcinolon-16α,17α-acetonid (Triamcinolonacetonid).

Spezielle Beispiele für Kombinationen von Wirksubstanzen sind:
- Kombinationen von Kortikosteroiden mit Antimykotika, Antibiotika oder Antiseptika. Eine bevorzugte Kombination ist z.B. (±)-cis-1-Acetyl-4- {4-([2-(2,4-dichlorphenyl)-2-(1H-imidazol-1-ylmethyl)-1,3-dioxolan-4-yl]methoxy)phenyl}-piperazin (Ketoconazol) und 11β,16α,17α, 21-Tetrahydroxy-1,4-pregnadien-3,20-dion-16,17-acetonacetal (Desonid).
- Kombinationen von Antimykotika synthetischer Herkunft mit Antimykotika natürlicher Herkunft. Eine bevorzugte Kombination ist bis-Phenyl-(2-chlor-phenyl)-1-imidazolylmethan (Clotrimazol) mit Teebaumöl.
- Kombinationen von verschiedenen Antimykotika natürlicher Herkunft. Eine bevorzugte Kombination ist Lavendelöl, Teebaumöl und Australian- Blue- Cypress- Oil.

Als geeignete pflegende Wirksubstanzen kommen gemäss vorliegender Erfindung vor allem Nähr- und Aufbaustoffe in Betracht. Bevorzugt sind Nähr- und Aufbaustoffe, die aus den Gruppen der Aminosäuren, der Vitamine und der Mineralstoffe ausgewählt sind.

Bevorzugte Aminosäuren sind (S)-2,6-Diaminohexansäure (Lysin), (R)-2-Amino-3-mercaptopropionsäure (Cystein) und insbesondere 2-Pyrrolidincarbonsäure (L-Prolin). Mit L-Prolin wurde ein Aufbaustoff gefunden, der sich zum Nagelaufbau und zur Nagelpflege als besonders geeignet erwiesen hat. L-Prolin wurde bisher lediglich als fakultativer Bestandteil von kosmetischen Mitteln zur Nagelpflege erwähnt, die als wirksame Komponente eine sulfurierte Aminosäure oder ein Derivat der sulfurierten Aminosäure enthalten (EP-A-0 534 810). Bevorzugte Vitamine sind Cis-2-(4-Carboxybutyl)-3,4-ureidotetrahydrothiophen (Biotin), (±)-2,4-Dihydroxy-N-(3-hydroxypropyl)-3,3-dimethylbutyramid (Panthenol), D(+)-2,4-Dihydroxy-N-(3-hydroxypropyl)-3,3-dimethylbutyramid (Dexpanthenol).

Bevorzugte Mineralstoffe sind anorganische und organische Calcium-, Magnesium- und Zinkverbindungen, vorzugsweise als organische Salze wie z.B. als Glycerophosphate oder Lactate.

Spezielle Kombinationen von Nähr- und Aufbaustoffen sind:

Kombinationen von 2-Pyrrolidincarbonsäure (L-Prolin) mit einem oder mehreren weiteren Nähr- und Aufbaustoffen, die aus den Gruppen der Aminosäuren, der Vitamine und der Mineralstoffen ausgewählt sind. Besonders bevorzugt sind Kombinationen von 2-Pyrrolidincarbonsäure (L-Prolin) mit einem oder mehreren Nähr- und Aufbaustoffen, die aus der aus (S)-2,6-Diaminohexansäure (Lysin), (R)-2-Amino-3-mercaptopropionsäure (Cystein), Gelatine, Cis-2-(4-Carboxybutyl)-3,4-ureidotetrahydrothiophen (Biotin), (±)-2,4-Dihydroxy-N-(3-hydroxypropyl)-3,3-dimethylbutyramid (Panthenol), D(+)-2,4-Dihydroxy-N-(3-hydroxypropyl)-3,3-dimethylbutyramid (Dexpanthenol) und anorganische oder organische Calcium-, Magnesium- oder Zinkverbindungen ausgewählt sind.

Die erfindungsgemässen topisch anwendbaren Mittel können neben einer oder mehrerer Wirksubstanzen und einem oder mehreren Verbindungen der Formel I übliche physiologisch verträgliche Hilfsstoffe enthalten. Geeignete Hilfsstoffe und Lösungsvermittler dieser Art sind beispielsweise Terpene oder Terpene enthaltende Öle, Alkohole, Ketone, Fettsäureester, Polyglykole, Tenside, Harnstoff, Antioxidantien und Komplexierungsmittel.

Geeignete Terpene sind acyclische, monocyclische und bicyclische Terpene sowie Öle, die diese Terpene enthalten. Beispiele für acyclische Terpene sind acyclische Terpenkohlenwasserstoffe, wie z.B. Myrcen, acyclische Terpenalkohole, wie z.B. Citronellol und Geraniol, sowie acyclische Terpenaldehyde und -ketone, wie z.B. Citral, α-Jonon und β-Jonon. Beispiele für monocyclische Terpene sind monocyclische Terpenkohlenwasserstoffe, wie z.B. α-Terpinen, γ-Terpinen und Limonen, monocyclische Terpenalkohole, wie z.B. Thymol, Menthol, Cineol und Carvacrol, sowie monocyclische Terpenketone, wie z.B. Menthon und Carvon. Beispiele für bicyclische Terpene sind Terpene aus der Carangruppe, wie z.B. Caron, Terpene aus der Pinangruppe, wie z.B. α-Pinen und β-Pinen, sowie Terpene aus der Bornangruppe, wie z.B.Campher und Borneol. Besonders geeignete Terpene sind monocyclische Terpenalkohole, wie z.B. Thymol und Menthol. Beispiele für geeignete Öle, die Terpene enthalten, sind Pfefferminzöl, Cardamomenöl, Geraniumöl, Rosenöl, Thujaöl und Thymianöl. Besonders geeignete Öle sind Pfefferminzöl, Lavendelöl und Thymianöl.

Geeignete Alkohole sind verzweigte oder unverzweigte Alkohole mit 1 bis 3 Hydroxygruppen und 2 bis 4 Kohlenstoffatomen, wobei die Hydroxygruppen teilweise oder vollständig verethert und verestert sein können. Speziell geeignete Alkohole sind Ethanol, 1-Propanol, 2-Propanol (Isopropanol), 1,2-Propandiol (Propylenglykol), 2-Phenylethanol (Phenylethylalkohol), 1-Butanol (Butylalkohol), Ethylenglycolmonomethylether (Methoxyethanol), Ethylenglycolmonophenylether (Phenoxyethanol), 1,2,3-Trihydroxypropan (Glycerin), Ethylacetat, Butylacetat, Glycerindiacetat (Diacetin) und Glycerintriacetat (Triacetin).

Als geeignete Ketone kommen beispielsweise Aceton und Methylethylketon
(2-Butanon) in Betracht.

Als Fettsäureester sind Ester von gesättigten und ungesättigten, verzweigten und unverzweigten Fettsäuren mit 8 bis 21 Kohlenstoffatomen geeignet, wobei die Alkoholkomponente verzweigte und unverzweigte Alkohole mit 1 bis 6 Kohlenstoffatomen umfasst. Speziell geeignete Fettsäureester sind Tridecancarbonsäureisopropylester, Tetradecancarbonsäureisopropylester (Isopropylmyristat), Pentadecancarbonsäuremethylester und 9-Octadecensäureglycerinmonoester (Glycerinmonooleat).

Ein geeignetes Polyglykol ist beispielsweise Polyethylenglykol 400.

Geeignete Tenside sind beispielsweise nicht ionogene oberflächenaktive Substanzen. Speziell geeignete Tenside sind Partialfettsäureester des Sorbitans (Span), Partialfettsäureester des Polyoxyethylensorbitans (Tween), Fettsäureester des Polyoxyethylens (Myrj) und Fettalkoholether des Polyoxyethylens (Brij).

Geeignete Antioxidantien sind beispielsweise Butylhydroxytoluol (BHT), Butyl-4-methoxyphenol (BHA), Tocopherole und Ascorbate.

Als Komplexierungsmittel eignen sich beispielsweise Ethylendiamintetraessigsäure (EDTA) und Dinatrium-Ethylendiamintetraessigsäure (Na₂-ETDA).

Als erfindungsgemässe topisch anwendbare Mittel kommen zum Beispiel Lösungen, Tinkturen, Emulsionen, Gele, Salben (Cremes) und Pasten in Betracht. Bevorzugte topische Darreichungsformen sind Lösungen. Bei der Herstellung von Lösungen erfordern manche Wirkstoffe, z.B. Prolin, zur Erhöhung ihrer Löslichkeit den Zusatz einer gewissen Menge Wasser in Verbindung mit einem Lösungsvermittler. Als Lösungsvermittler sind niedere Alkanole, wie Methanol, Ethanol, Propanol und Isopropanol, sowie Aceton geeignet.

Die erfindungsgemässen topisch anwendbaren Mittel können beispielsweise in der Weise hergestellt werden, dass man die einzelnen Komponenten homogen mischt und die Mischung gegebenenfalls unter Erwärmen (maximal 80°C) solange rührt, bis eine homogene Wirkstofflösung entstanden ist. Die erhaltene Lösung wird bevorzugt direkt als solche zur topischen Applikation verwendet. Die Lösung kann aber auch unter Zugabe von weiteren physiologisch verträglichen Formulierungshilfsstoffen mittels konventionellen Lösungs-, Misch- und Suspendierverfahren in eine andere topische, bereits aufgeführte Darreichungsform gebracht werden.

Bevorzugt werden die erfindungsgemässen topisch anwendbaren Mittel in Form von Lösungen verwendet. Bevorzugte topisch anwendbare Mittel enthalten gemäss vorliegender Erfindung
0,1 bis 20 Gew.-% einer oder mehrerer Wirksubstanzen,
1 bis 99,90 Gew.-% einer oder mehrerer Verbindungen der Formel I und
0 bis 98,90 Gew.-% eines oder mehrerer physiologisch verträglicher Hilfsstoffe.

Die Erfindung betrifft weiterhin die Verwendung der erfindungsgemässen topisch anwendbaren Mittel zur Behandlung, Prävention, Nachbehandlung oder unterstützenden Behandlung von Nagelerkrankungen und periungualen Erkrankungen sowie zur Nagelpflege. Ausserdem betrifft die vorliegende Erfindung die Verwendung der erfindungsgemässen Mittel zur Behandlung von Pilzinfektionen von Hufen, Klauen und Krallen von Haus- und Nutztieren.

Topisch anwendbare Mittel enthaltend Antimykotika eignen sich beispielsweise für folgende Indikationen:
- Behandlung, Prävention und Nachbehandlung von Onychomykosis, verursacht durch Dermatophyten, Hefen oder Schimmelpilze oder Mischinfektionen
- Behandlung, Prävention und Nachbehandlung von Nagelpilzinfektionen bei Patienten mit Psoriasis, Diabetes oder AIDS
- Unterstützung der Behandlung von periungualen Nagelinfektionen, wie z.B. Candida paronychium.

Topisch anwendbare Mittel enthaltend Antibiotika eignen sich beispielsweise für folgende Indikationen:
- Unterstützung der Behandlung und/oder Prävention von Nagel- und periungualen Infektionen, verursacht durch Bakterien.

Topisch anwendbare Mittel enthaltend Antiseptika eignen sich beispielsweise für folgende Indikationen:
- Behandlung und Prävention von Nagel- und periungualen Infektionen, verursacht durch unspezifische oder nicht identifizierte Erreger.

Topisch anwendbare Mittel enthaltend Kortikosteroide oder Kombinationen von Kortikosteroiden mit Antimykotika, Antibiotika oder Antiseptika eignen sich beispielsweise für folgende Indikationen:
- Behandlung, Prävention, Nachbehandlung oder unterstützende Behandlung von Nagel-Psoriasis oder anderen entzündlichen Nagel- und periungualen Zuständen.

Die erfindungsgemässen pharmazeutischen, topisch anwendbaren Mittel eignen sich zur Behandlung von Nagelerkrankungen und periungualen Erkrankungen an Zehen- und Fingernägeln, sowie zur Behandlung von Erkrankungen der Hufe, Klauen und Krallen von Haus- und Nutztieren. Die Applikationsfrequenz der pharmazeutischen Mittel richtet sich nach dem Ausmass und der Lokalisation der Erkrankung. Im allgemeinen genügt eine ein- bis dreimalige Anwendung pro Tag. Die Lösung wird hierbei direkt auf den kranken Nagel bezw. den Huf, die Klaue oder die Kralle und bei Bedarf auf die ebenfalls betroffenen umliegenden Hautbezirke aufgetragen. Die Therapie sollte nachdem im Labortest Pilze, Sporen oder andere Erreger nicht mehr nachweisbar sind noch etwa 2-4 Wochen weitergeführt werden, um einen Rückfall zu verhindern.

Die erfindungsgemässen kosmetischen, topisch anwendbaren Mittel, enthaltend einen oder mehrere Nähr- und Aufbaustoffe, eignen sich zur Nagelpflege, wie zum Beispiel bei Nagelatrophien an Zehen- und Fingernägeln. Nagelatrophien beinhalten beispielsweise brüchige, spröde und dünne Nägel sowie punktförmige oder streifige weisse Flecken. Die Zubereitung wird auf den oder die kosmetisch unschönen Nägel und bei Bedarf auch auf umliegende Hautbezirke aufgetragen. Die Applikationsfrequenz der Zubereitung richtet sich nach dem Ausmass und der Lokalisation der Atrophie. Im allgemeinen genügt eine ein- bis zweimalige Anwendung pro Tag.

Die erfindungsgemässen topisch anwendbaren Mittel haben den Vorteil, dass sie den erkrankten Nagel mitsamt den Wirksubstanzen innerhalb von wenigen Tagen vollständig durchdringen bzw. sättigen und im Nagelbett und an der Nagelwurzel ihre Wirkung entfalten können. Durch die bessere Penetration und den damit verbundenen rascheren Wirkungseintritt ist die Behandlung von Nagelkrankheiten in der Regel nach etwa zwei bis vier Monaten abgeschlossen. Dadurch wird die Patienten-Compliance deutlich verbessert, da die bei anderen Behandlungsmethoden erforderliche lange Behandlungsdauer wesentlich verkürzt wird. Bei erkrankter Haut, insbesondere bei periungualen Hautbezirken, tritt der Heilungsprozess beziehungsweise der Pflegeeffekt rascher ein, da der Wirkstoff effizient und schnell in die Haut eindringt. Die Nagelpflege sollte in der Regel einen Monat lang durchgeführt werden. Zur Erhaltung der gesunden Nagelsubstanz kann das Nagelpflegeprodukt auch über einen längeren Zeitraum angewendet werden.

Die vorliegende Erfindung wird durch folgende Beispiele veranschaulicht:

### Beispiel 1: Australian-Blue-Cypress -Oil -Teebaumöl- Lavendelöl-Lösung 6%

| | |
|---|---|
| Australian-Blue-Cypress -Oil | 2,0 g |
| Teebaumöl | 2,0 g |
| Lavendelöl | 2,0 g |
| Isoamylacetat | 94,0 g |

Die Mischung wird solange gerührt, bis eine homogene Lösung entstanden ist.

### Beispiel2: Prolin-Lösung 2.0 %

| | |
|---|---|
| L- Prolin | 2,0 g |
| Wasser (deionisiert) | 2,0 g |
| Ethanol | 48,0 g |
| Amylacetat | 48,0 g |

L-Prolin wird in Ethanol und Wasser unter Rühren gelöst. Anschliessend wird Amylacetat beigefügt und solange weitergerührt, bis eine homogene Lösung entstanden ist.

### Beispiel 3: Amorolfin-Lösung 1%

| | |
|---|---|
| Amorolfin | 1,0 g |
| Ethanol | 10,0 g |
| 1- Hexylacetat | 89,0 g |

Amorolfin wird unter Rühren in die Mischung von Ethanol und Hexylacetat eingetragen und solange gerührt bis eine homogene Lösung entstanden ist.

### Beispiel 4: Terbinafin-Lösung 1%

| | |
|---|---|
| Terbinafin Base | 1,0 g |
| Isoamylacetat | 99 g |

Die Substanzen werden in ein Becherglas eingewogen und solange gerührt, bis eine homogene Lösung entstanden ist

### Beispiel 5: Creme mit Amylacetat

| | |
|---|---|
| H2O ionenfrei | 720 g |
| Carbopol Ultrez 10 | 9,6 g |
| Glycerin | 24 g |
| Sonnenblumenöl | 216 g |
| Emulgade 1000 NI | 45,6 g |
| Lanette N | 9,6 g |
| Amylester | 36 g |
| Phenonip | 9,6 g |
| Triethanolamin | |

H2O wird vorgelegt, unter Rühren Carbopol aufgestreut und quellen lassen. Glycerin zugeben und auf+50°C erwärmt.

Aus Sonnenblumenöl, Emulgade und Lanette wird unter Rühren und Erwärmen auf 70°C eine klare Lösung hergestellt.
Amylester wird zur Fettphase gegeben und das Gemisch unter kräftigem Rühren mit der Wasserphase homogenisiert.
Phenonip wird eingearbeitet.
Die Creme abkühlen lassen und mit Triethanolamin auf pH 5.5 eingestellt.

## Patentansprüche

1. Verwendung von einer oder mehreren Verbindungen der Formel I
R-O-R₁ (I)
in welcher
R einen geradkettigen oder verzweigten Alkylrest mit 5 - 8 Kohlenstoffatomen, und
R₁ eine Formylgruppe oder eine Acetylgruppe bedeutet,
zur Verbesserung des Penetrationsvermögens von einer oder mehreren pflegenden Wirksubstanzen bei Anwendung in Form eines topischen Mittels zur Nagelpflege, wobei das Mittel gegebenenfalls physiologisch verträgliche Hilfstoffe enthält.

2. Verwendung von einer oder mehreren Verbindungen der Formel I
R-O-R₁ (I)
in welcher
R einen geradkettigen oder verzweigten Alkylrest mit 5-8 Kohlenstoffatomen, und
R₁ eine Formylgruppe oder eine Acetylgruppe bedeutet,
zur Herstellung eines topisch anwendbaren Mittels zur Behandlung von Nagelerkrankungen, wobei die Verbindungen der Formel (I) zur Verbesserung des Penetrationsvermögens von einer oder mehreren enthaltenen therapeutischen Wirksubstanzen zugesetzt werden, und wobei das Mittel gegebenenfalls physiologisch verträgliche Hilfsstoffe enthält.

3. Verwendung nach Asprüchen 1 und 2, **dadurch gekennzeichnet, dass** man als Lösungsvermittler der Formel (I) Amylformiat oder auch Amylacetat verwendet.

4. Verwendung nach Ansprüchen 1 oder 2, **dadurch gekennzeichnet, dass** man als Lösungsvermittler der Formel (I) 1-Hexylformiat, oder 1-Hexylacetat verwendet.

5. Verwendung nach Ansprüchen 1 und 2, **dadurch gekennzeichnet, dass** man als Lösungsvermittler der Formel (I) 1-Heptylformiat oder 1-Heptylacetat verwendet.

6. Verwendung nach Ansprüchen 1 und 2, **dadurch gekennzeichnet, dass** man als Lösungsvermittler der Formel (I) 2-Heptylformiat oder 2-Heptylacetat verwendet.

7. Verwendung nach Ansprüchen 1 und 2, **dadurch gekennzeichnet, dass** man als Lösungsvermittler der Formel (I) 1-Octylformiat oder 1-Octylacetat verwendet.

8. Verwendung nach Ansprüchen 1 und 2, **dadurch gekennzeichnet, dass** man als Lösungsvermittler der Formel (I) 2-Octylformiat oder 2-Octylacetat verwendet.

9. Verwendung nach Ansprüchen 1 und 2, **dadurch gekennzeichnet, dass** man einen oder mehrere Hilfsstoffe aus der Gruppe, die Terpene oder Terpene enthaltende Oele, Alkohole, Ketone, Fettsäureester, Polyglykolen, Tenside, Harnstoff, Antioxydantien und Komplexierungsmittel enthält, verwendet.

10. Verwendung nach Anspruch 2, **dadurch gekennzeichnet dass** man eine oder mehrere therapeutische, aus der aus Antimykotika synthetischer und natürlicher Herkunft, Antibiotika, Antiseptika, und Kortikosteroiden bestehenden Gruppe ausgewählte Wirksubstanzen enthält, verwendet.

## Claims

1. Utilization of one or more compounds of the formula I
R-Q-R₁ (I)
where
R is a straight-chained or branched alkyl radical with 5-8 carbon atoms and
R₁ is a formyl group or an acetyl group,
to improve the penetrability of one or more active therapeutic substances during the application under the form of a topical nail-care product that may contain physiologically tolerated excipients.

2. Utilization of one or more compounds of the formula I
R-O-R₁ (I)
where
R is a straight-chained or branched alkyl radical with 5 - 8 carbon atoms and
R₁ is a formyl group or an acetyl group,
for the creation of a topically applicable product for the treatment of nail diseases, where the compounds of the formula (I) are added to improve the penetrability of one or more of the active therapeutic substances and where the product may contain physiologically tolerated excipients.

3. Utilization according to claims 1 and 2, **characterized by** the use of amyl formate or amyl acetate as a solubilising agent for the formula (I)

4. Utilization according to claims 1 and 2, **characterized by** the use of 1-hexyl formate or 1-hexyl acetate as a solubilising agent for the formula (I)

5. Utilization according to claims 1 and 2, **characterized by** the use of 1-heptyl formate or 1-heptyl acetate as a solubilising agent for the formula (I).

6. Utilization according to claims 1 and 2, **characterized by** the use of 2-heptyl formate or 2-heptyl acetate as a solubilising agent for the formula (I).

7. Utilization according to claims 1 and 2, **characterized by** the use of 1-octyl formate or 1-octyl acetate as a solubilising agent for the formula (I).

8. Utilization according to claims 1 and 2, **characterized by** the use of 2-octyl formate or 2-octyl acetate as a solubilising agent for the formula (I).

9. Utilization according to claims 1 and 2, **characterized by** the use of one or more excipients selected from the group containing terpene or terpene oils, alcohols, ketones, ester fatty acids, polyglycols, tensides, urea, antioxidants and complexation agents.

10. Utilization according to claim 2, **characterized by** the use of one or more active therapeutic substances selected from the group composed of antimycotics of natural or synthetic origin, antibiotics, antiseptics and corticosteroids.

## Revendications

1. Utilisation d'un ou plusieurs des composés de la formule I
R-O-R₁ (I)
où
R est un radical alkyle linéaire ou ramifié avec 5-8 atomes de carbone et
R₁ est un groupe formyle ou acétyle,
pour améliorer la capacité de pénétration d'une ou plusieurs substances thérapeutiques actives lors de l'application sous forme d'un produit topique pour le soin des ongles, contenant, le cas échéant, des excipients tolérés physiologiquement.

2. Utilisation d'un ou plusieurs des composés de la formule I
R-Q-R₁ (I)
où
R est un radical alkyle linéaire ou ramifié avec 5-8 atomes de carbone et
R₁ est un groupe formyle ou acétyle,
pour la fabrication d'un produit appliqué topiquement, pour le traitement des maladies des ongles, dans lequel les composés de la formule (I) sont ajoutés pour améliorer la capacité de pénétration d'une ou plusieurs des substances thérapeutiques actives et où le produit contient, le cas échéant, des excipients tolérés physiologiquement.

3. Utilisation conformément aux revendications 1 et 2, **caractérisée par** l'utilisation de formiate d'amyle ou d'acétate d'amyle comme agent solubilisant de la formule (I).

4. Utilisation conformément aux revendications 1 et 2, **caractérisée par** l'utilisation de formiate de 1-hexyle ou d'acétate de 1-hexyle comme agent solubilisant de la formule (I).

5. Utilisation conformément aux revendications 1 et 2, **caractérisée par** l'utilisation de formiate de 1-heptyle ou d'acétate de 1-heptyle comme agent solubilisant de la formule (I).

6. Utilisation conformément aux revendications 1 et 2, **caractérisée par** l'utilisation de formiate de 2-heptyle ou d'acétate de 2-heptyle comme agent solubilisant de la formule (I).

7. Utilisation conformément aux revendications 1 et 2, **caractérisée par** l'utilisation de formiate de 1-octyle ou d'acétate de 1-octyle comme agent solubilisant de la formule (1).

8. Utilisation conformément aux revendications 1 et 2, **caractérisée par** l'utilisation de formiate de 2-octyle ou d'acétate de 2-octyle comme agent solubilisant de la formule (I).

9. Utilisation conformément aux revendications 1 et 2, **caractérisée par** l'utilisation d'un ou plusieurs excipients sélectionnés dans le groupe comprenant des terpènes ou des huiles contenant des terpènes, des alcools, des cétones, des esters d'acides gras, des polyglycoles, des tensides, de l'urée, des antioxydants et des agents de complexion.

10. Utilisation conformément à la revendication 2, **caractérisée par** l'utilisation d'une ou plusieurs substances thérapeutiques actives, sélectionnées dans le groupe comprenant des antimycotiques d'origine synthétique ou naturelle, des antibiotiques, des antiseptiques et des corticostéroïdes.
